# EUROPEAN PATENT SPECIFICATION

(11) **EP 3 620 508 B1**
(45) Date of publication and mention of the grant of the patent: **28.04.2021**
(21) Application number: 19195328.0
(22) Date of filing: 04.09.2019
(51) Int. Cl.: C12M 3/06, C12M 1/12, C12M 1/42

(54) **MICROFLUIDIC DEVICE FOR ELECTRICAL MEASUREMENT AND/OR STIMULATION**
MIKROFLUIDISCHE VORRICHTUNG ZUR ELEKTRISCHEN MESSUNG UND/ODER STIMULATION
DISPOSITIF MICROFLUIDIQUE DE MESURE ET/OU DE STIMULATION ÉLECTRIQUES

(30) Priority: 04.09.2018 IT 201800008340
(43) Date of publication of application: 11.03.2020
(73) Proprietor: BIOMIMX S.r.l., 20158 Milano (IT)
(72) Inventor: RASPONI, Marco, 20133 MILANO (IT); VISONE, Roberta, 20133 MILANO (IT); UGOLINI, Giovanni Stefano, 20133 MILANO (IT)
(74) Representative: Rigamonti, Dorotea

(56) References cited:
- WO-A2-2011/121427
- US-A1- 2006 160 221
- ANDREA PAVESI ET AL: "How to embed three-dimensional flexible electrodes in microfluidic devices for cell culture applications", LAB ON A CHIP, vol. 11, no. 9, 1 January 2011 (2011-01-01), page 1593, XP055582731, ISSN: 1473-0197, DOI: 10.1039/c1lc20084d
- ANDREA PAVESI ET AL: "Controlled electromechanical cell stimulation on-a-chip", SCIENTIFIC REPORTS, vol. 5, 2 July 2015 (2015-07-02), page 11800, XP055255659, DOI: 10.1038/srep11800
- VAN DER HELM MARINKE W ET AL: "Direct quantification of transendothelial electrical resistance in organs-on-chips", BIOSENSORS AND BIOELECTRONICS, ELSEVIER SCIENCE LTD. UK, AMSTERDAM, NL, vol. 85, 8 June 2016 (2016-06-08), pages 924-929, XP029680676, ISSN: 0956-5663, DOI: 10.1016/J.BIOS.2016.06.014
- GRIEP L M ET AL: "BBB ON CHIP: microfluidic platform to mechanically and biochemically modulate blood-brain barrier function", BIOMEDICAL MICRODEVICES, KLUWER ACADEMIC PUBLISHERS, BO, vol. 15, no. 1, 6 September 2012 (2012-09-06), pages 145-150, XP035164565, ISSN: 1572-8781, DOI: 10.1007/S10544-012-9699-7
- Shuichi Takayama ET AL: "Topographical Micropatterning of Poly(dimethylsiloxane) Using Laminar Flows of Liquids in Capillaries", Adv. Mater., 13(8), 1 January 2001 (2001-01-01), pages 391-241, XP055583185, Retrieved from the Internet: URL:https://onlinelibrary.wiley.com/doi/ab s/10.1002/1521-4095%28200104%2913%3A8%3C57 0%3A%3AAID-ADMA570%3E3.0.CO%3B2-B [retrieved on 2019-04-24]

## Description

### Technical Field

The subject matter of the present invention is a microfluidic device (10) for the generation and/or cultivation and/or maturation and/or analysis of a cell matrix where said microfluidic device (10) comprises:
- a confinement means (A) that defines at least one compartment that is a growth chamber (7) adapted to contain a cell matrix;
- at least one guide (1, 2, 3, 4, 73) for housing and guiding the insertion of at least one electrode (20), where the volume of said at least one guide is afforded inside said confinement means (A);
characterised in that said at least one guide (1, 2, 3, 4, 73) is separated from said at least one growth chamber (7) by a wall (6), where said wall (6) is made of a material that is perforable and has a thickness in the range 100 µm - 5 mm, or 150 µm - 1.5 mm, or 200 µm - 1 mm.

### Prior Art

Electrical measurements and/or stimulations are fundamentally important for monitoring key parameters in *in vitro* systems, in basic and translational applications. By way of example, electrical measurements are used for electrophysiology studies, e.g. cardiac electrophysiology, measurement of neuron activity. Another application is in the measurement of the formation of the endothelial/epithelial barrier *in vitro* (TEER) or in electrical stimulation *in vitro,* by way of example in the stimulation of cardiac tissue or skeletal muscle tissue.

The gold standard for electrical measurements *in vitro* is currently comprised of MEA systems (array of microelectrodes) characterised by a monolayer cell culture on planar electrodes.

Alternatively, patch-clamp techniques are used, where micro-manipulated electrodes can perforate and measure the electrical activity in single cells.

The advancement of tissue engineering, along with the availability of 3D cell cultures and microfluidic methodologies, has not been followed by an equally effective development of technologies that allow electrical measurements and/or stimulations to be performed effectively *in vitro.*

The coupling of physical electrodes with microfluidic devices is currently only possible in two configurations:
i) electrodes inserted in the microfluidic device in the manufacturing step and constantly in contact with the growth chamber/biological material;
ii) electrodes inserted manually as required through access ports to the microchannels.
The first method poses problems of sterility, bio-compatibility of materials and manufacturing complexity; the second method envisages manual and therefore imprecise and not very reproducible positioning of the electrodes in wells that are generally distant from the biological material, therefore introducing problems related to poor reproducibility, loud electrical noise etc.
CN103789206 describes a system applicable to 2D cell cultures only, where the electrodes are inserted manually and therefore without the necessary precision.
CN2795855U describes a microfluidic device that comprises a channel for the measurement of interest that is open at the side. Although the presence of an open measurement channel makes it easy to access through a microelectrode, in order to prevent fluid leaks and/or guarantee control of the flow, it needs to be coupled and continuously positioned over time to a sealing system subject to alignment. The solution therefore imposes the need for fine tuning of the position of the electrode from the outside.
Andrea Pavesi et al., in "How to embed three-dimensional flexible electrodes in microfluidic devices for cell culture applications" Lab on a Chip, 9, 2011, describe a microfluidic device comprising a thin wall made of PMDS and having a thickness of 30 µm that separates the electrode channels from the growth chamber. The electrode is exposed to the growth chamber after chemical etching of the thin wall. Although in the described solution the electrodes are brought in contact with the growth chamber, they cannot touch directly nor impale a 3D cell matrix.

There is therefore a strongly felt need for a device and a method adapted to allow effective electrical measurements in the complex *in vitro* models currently available.

### Object of the invention

The subject matter of the present invention is a microfluidic device that enables effective electrical measurements and/or stimulations.

Further subject matter of the present invention is a kit for effective electrical measurements and/or stimulations in microfluidic conditions.

Further subject matter of the present invention is a method for electrical measurements and/or stimulations in a microfluidic device.

The characteristics of said microfluidic device, illustrated in different embodiments of the following examples, are contained in the claims.

### Brief description of the drawings

FIGURE 1: Microfluidic device a) wall not perforated; b) wall perforated.
FIGURE 2: An embodiment of the microfluidic device a) top view; b) perspective view of the explosion of the microfluidic device, which highlights the components thereof.
FIGURE 3: A further embodiment of the device a) top view; b) perspective view of the components of the device.
FIGURE 4: A further embodiment of the device a) top view; b) lateral sectional view of the explosion of the microfluidic device, which highlights the components thereof.
FIGURE 5: Analysis of the electrical activity of cardiac cell constructs after treatment with a) Sotalol, b) Verapamil.
FIGURE 6: Impedance variation when measured in the presence of a cell barrier through a) voltmeter or b) impedance meter.
FIGURE 7: A further embodiment of the device a) top view; b) perspective view of the components of the device.

### Detailed description of preferred embodiments of the invention

### Definitions:

"Microfluidic device" means herein a system provided with at least one growth chamber and/or at least one channel for the confinement and/or movement of fluids in microscale, i.e. volumes of fluids comprised between femtolitres and hundreds of microlitres.

The expression "cell matrix" means herein a cell culture, a three-dimensional tissue construct, and/or any intermediate stage thereof such as, for example, a 2D cell construct or a three-dimensional cell construct.

The expression "three-dimensional cell construct" means herein a set of cells structurally connected to one another, either directly or through a polymeric structure. Such structure may be of natural or synthetic origin, or a combination thereof.

The expression "three-dimensional tissue construct" means herein a set of cells functionally connected to one another, able to respond not only individually but also as a functional syncytium.

The expression "cell culture" means herein a suspension or aggregate of cells in a medium kept alive and grown outside their native environment.

The expression "controlled generation and/or cultivation and/or maturation" means herein the stages of the transformation process of an initial cell culture in a three-dimensional cell and/or tissue construct through a device and a process in which the stimuli adapted to promote it can be regulated and repeated.

Preferably, the cell matrix comprises a natural or synthetic polymer, or a combination thereof, more preferably a fluid polymeric solution and/or a gel, even more preferably a hydrogel.

Preferably, the fluid polymeric solution comprises the polymers selected in the group that comprises fibrin, collagen, hyaluronic acid, elastin, fibroin, agarose, chitosan, alginate and the like, and mixtures thereof, more preferably fibrin.

Preferably, the hydrogel is selected in the group that comprises polyethylene glycol (PEG), fibrin gel, collagen, gelatine, hyaluronic acid, elastin, fibroin, agarose, chitosan, alginate and the like, and combinations thereof, more preferably fibrin gel.

The term "microfluidic channel" means herein a conduit adapted to confine a fluid and having at least one dimension comprised between 1 µm and 1000 µm. Unless otherwise indicated, it is to be understood that the shape of the perimeter of the microfluidic channel is substantially rectangular.

Preferably, the length or the width of the microfluidic channel corresponds to the length or to the width of the growth chamber.

Preferably, the microfluidic channel has a width comprised between 20 µm and 2000 µm, more preferably comprised between 100 µm and 500 µm, even more preferably 300 µm; and a length comprised between 50 µm and 20 mm, more preferably comprised between 200 µm and 10 mm, even more preferably of about 7.5 mm.

"Confinement means" defines herein at least one element that delimits in the microfluidic device at least one compartment that is a growth chamber and/or at least one microfluidic channel and/or at least one guide.

The term "hydrophobic" in relation to the confinement means is used herein to indicate that it is made of a material that is hydrophobic *per se* or rendered so through a chemical and/or physical surface treatment.

Preferably, the confinement means is made of a chemically inert material, more preferably selected from silicone rubber, e.g. polydimethylsiloxane (PDMS), fluorinated rubber, polystyrene (PS), polymethylmethacrylate (PMMA), polycarbonate (PC), glass, silicon, polyethylene glycol (PEG) and the like, and combinations thereof, more preferably PDMS.

In one embodiment, the confinement means comprises a plurality of micropillars. Preferably, the micropillars are arranged in rows, more preferably at a distance between adjacent micropillars comprised between 5 µm and 250 µm, more preferably comprised between 15 µm and 100 µm, even more preferably about 50 µm. Preferably, adjacent micropillars are equidistant from one another. Preferably the micropillars are pillars with a section having any shape, preferably with a polygonal or circular section with height dimensions comprised between 20 µm and 400 µm, more preferably comprised between 80 µm and 200 µm, even more preferably of about 100 µm, and a radius or a side of the polygon comprised between 5 µm and 100 µm, more preferably comprised between 20 µm and 60 µm, even more preferably of about 30 µm.

Preferably, the micropillars have the same dimensions as one another.

Preferably, the length of a row of micropillars is comprised between 50 µm and 20 mm, more preferably between 200 µm and 10 mm, more preferably of about 7.5 mm. Preferably, the micropillars are arranged in two parallel rows so as to define a microfluidic channel. Preferably, the distance between two rows of parallel micropillars, i.e. the width of the microfluidic channel that they define, is comprised between 100 µm and 2000 µm, more preferably comprised between 200 µm and 400 µm, even more preferably about 300 µm. In one embodiment, said micropillars are T-shaped micropillars.

Preferably, a microfluidic channel is split into a plurality of microfluidic channels in series.

Preferably, every part of the device is made of a chemically inert material, more preferably selected from silicone rubber, e.g. polydimethylsiloxane (PDMS), fluorinated rubber, polystyrene (PS), polymethylmethacrylate (PMMA), polycarbonate (PC), glass, silicon, polyethylene glycol (PEG), and combinations thereof, preferably PDMS.

"Perforable material" for the purpose of the present invention means a material that can be perforated by applying pressure with a sharp object that is an electrode with the characteristics described in the following paragraphs. The thickness of said perforable material is comprised in the range 100 µm - 5 mm, or 150 µm - 1.5 mm, or 200 µm - 1 mm.

Said perforable material is preferably selected in the group that comprises elastomers, silicone rubber, e.g. polydimethylsiloxane (PDMS), fluorinated rubber, polyurethanes, polystyrene (PS), polymethacrylate (PMMA), polycarbonate (PC), polyethylene glycol (PEG) and the like, and combinations thereof, more preferably PMDS. Advantageously, the microfluidic device comprises one or more openings for the injection of the cell matrix, as for the inlet and outlet of the growth medium.

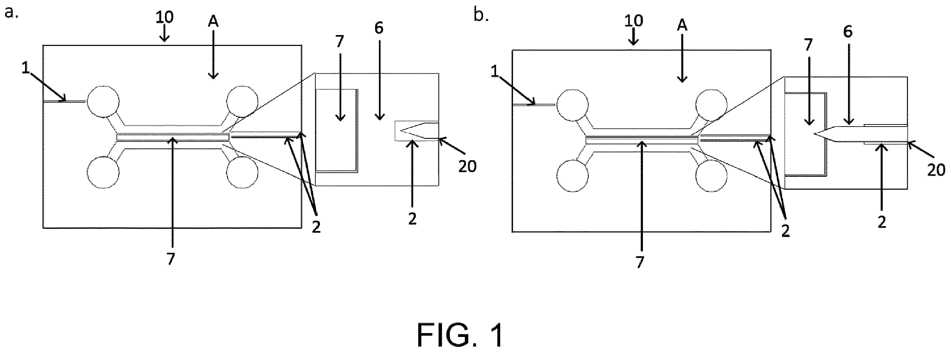

The subject matter of the present invention, with reference to figure 1, is a **microfluidic device (10)** suitable for the generation and/or cultivation and/or maturation and/or analysis of a cell matrix where said microfluidic device (10) comprises:
- a confinement means (A) that defines at least one compartment that is a growth chamber (7) adapted to contain a cell matrix;
- at least one guide (1, 2) for housing and guiding the insertion of microelectrodes, where the volume of said at least one guide is afforded inside said confinement means (A);
characterised in that said at least one guide (1, 2) is separated from said at least one growth chamber (7) by a wall (6), where said wall (6) is made of a perforable material.

According to the present invention, said wall (6) is perforable, i.e. said wall, at least in the area which has to be perforated by the electrode (20), has a thickness comprised in the range 100 µm - 5 mm, or 150 µm - 1.5 mm, or 200 µm - 1 mm.

The authors of the present invention have surprisingly shown that walls with a lower thickness than the one here described do not allow to solve the technical problem object of the present invention, that is they do not allow to accurately position in a tight manner an electrode inside the growth chamber. When using walls with a thickness lower than the range claimed here, when the same are perforated, the requested sealing is lost.

In fact, to maintain the tightness after perforation of the wall by an electrode, it is necessary that said wall is firmly anchored to the underlying substrate and said anchoring is the more critical the thinner the thickness of the wall itself. Therefore, silicone walls with a thickness lower than 100 µm and assembled by plasma treatment on another surface (for example, silicone, glass) do not appear to be leak-proof.

In a preferred embodiment, along said guide (1, 2) there are one or more additional walls, named support walls. Said support walls have independently the features described for the wall (6), i.e. they are made of a perforable material, having a thickness comprised in the range 100 µm - 5 mm. In this embodiment, the tightness of the device is increased.

Moreover, in the work configuration, the electrode housed in the guide is mechanically stabilized in the correct position by said support walls.

The panel 1a shows the microfluidic device in the rest configuration, where said wall (6) as can be seen from the insert with larger magnification, is not perforated. An electrode (20) is positioned in the guide (2). The panel 1b shows the microfluidic device in the work configuration, where said wall (6) as can be seen from the insert with larger magnification, has been perforated by the electrode (20). The electrode (20), housed in said guide (2), has reached the wall (6) and perforated it reaching the growth chamber (7) sliding in said guide (2). This means that in the microfluidic device according to the present invention the electrode is able to reach the growth chamber exactly in the desired position, where the direction of the movement of said electrode is defined precisely by the fact that it is housed and can slide inside said guide (2). Moreover, once the electrode reaches the growth chamber, the tightness of the growth chamber itself is maintained, thanks to the thickness of the wall perforated by the electrode.

In one embodiment, at least one portion of said wall (6) is perforated. Said wall (6) is preferably perforated in the proximal portion to said at least one guide and in the direction of the guide itself, so as to facilitate the reaching of said growth chamber (7) of said electrode (20) housed in said guide (2).

Even more preferably, when said wall is perforated it is perforated with an opening that has a smaller diameter than the diameter of the electrode that is to be inserted in the opening itself, so as to facilitate said insertion while keeping rigid the position constraints conferred to said electrode by said guide.

In one embodiment, said confinement means (A) is made, fully or partly, of the same material of which said wall (6) is made.

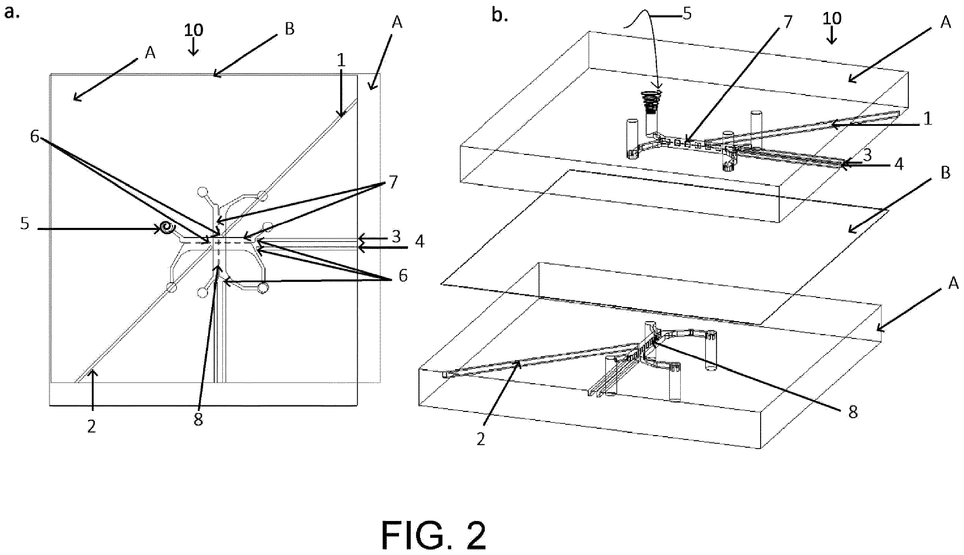

With reference to the embodiment of figure 2, said microfluidic device (10) comprises:
- two confinement means (A) that define at least one first compartment (7) and at least one second compartment (8);
- a membrane (B) interposed between said two confinement means (A);
- optionally, at least one reference electrode (5) housed in at least one of said confinement means (A);
- one or more guides (1, 2, 3, 4) for housing and guiding the insertion of microelectrodes, where the volume of said at least one guide is afforded inside said confinement means (A);
where said at least one guide (1, 2, 3, 4) is separated from said at least one compartment (7) by a wall (6), where said wall (6) is made of a material that is perforable and has a thickness comprised in the range 100 µm - 5 mm, or 150 µm - 1.5 mm, or 200 µm - 1 mm.

Said membrane (B) is a porous membrane; alternatively, it is an elastic membrane.

Said microfluidic device (10) can be applied for example in the generation and maturation of cell constructs, e.g. muscular constructs such as cardiac constructs. In this embodiment, said first compartment (7) is a growth chamber, said second compartment (8) is an actuation chamber and said membrane (B) is an elastic membrane.

Said microfluidic device (10) can further be applied for TEER measurements. In this embodiment, said at least one first compartment (7) and said at least one second compartment (8) are growth chambers and said membrane (B) is a porous membrane.

Said reference electrode, where provided, is for example an AgCI electrode.

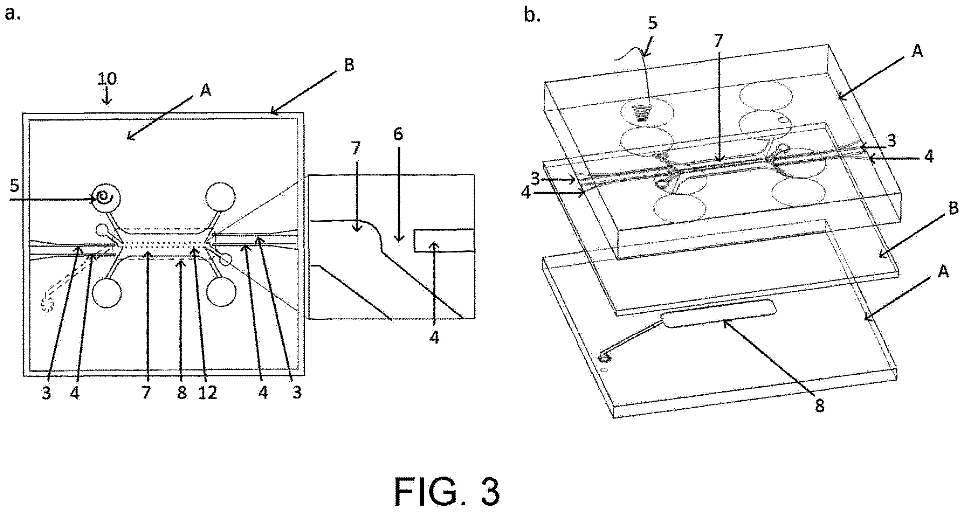

With reference to figure 3, said microfluidic device (10) comprises:
- two confinement means (A) that define at least one compartment that is a growth chamber (7) adapted to contain a cell matrix and at least one compartment that is an actuation chamber (8) adapted to provide a mechanical stimulation through pneumatic activation;
- a membrane (B) that is an elastic membrane interposed between said two confinement means (A), separating said growth chamber (7) from said actuation chamber (8);
- at least one reference electrode (5) housed in at least one of said confinement means (A);
- guides (3, 4) for housing and guiding the insertion of microelectrodes, where the volume of said guides is afforded inside said confinement means (A);
where said guides (3, 4) are separated from said at least one growth chamber (7) by a wall (6), where said wall (6) is made of a material that is perforable and has a thickness in the range 100 µm - 5 mm, or 150 µm - 1.5 mm, or 200 µm - 1 mm.

In this embodiment, which is shown to be particularly suitable for applications on cell matrices that comprise cardiac cells and/or tissues, said growth chamber (7) has an elongated shape and said guides (3, 4) reach said growth chamber at one or both of the ends of said growth chamber. Preferably, said guide (4) is positioned along the central axis (12) of said microfluidic device (10) and said guide (3) is parallel to said guide (4).

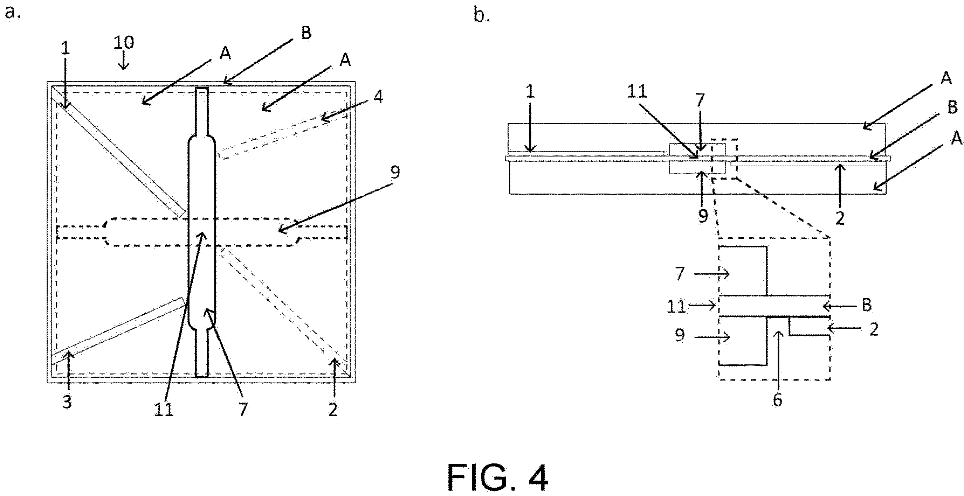

In the embodiment depicted in figure 4, said microfluidic device (10) comprises:
- two confinement means (A) that define at least one compartment that is a growth chamber (7) adapted to contain a cell matrix and at least one compartment that is a collection chamber (9);
- a membrane (B) interposed between said two confinement means (A) that separates said growth chamber (7) from said collection chamber (9), where said membrane (B) is a porous membrane;
- guides (1, 2) for housing and guiding the insertion of microelectrodes, where the volume of said guides is afforded inside said confinement means (A);
where said guides (1, 2) are separated from said at least one growth chamber (7) by a wall (6), where said wall (6) is made of a material that is perforable and has a thickness in the range 100 µm - 5 mm, or 150 µm - 1.5 mm, or 200 µm - 1 mm.

In this embodiment, which is particularly suitable for studies of transport through epithelial and/or endothelial barriers, as an example through the blood-brain barrier, said growth chamber (7) and said collection chamber (9) have an elongated shape and are arranged on top of one another, orthogonal to one another, so as to form a central exchange area (11). Said guides (1, 2) reach said growth chamber (7) and said collection chamber (9), respectively, in said central exchange area (11).

As an example, in said growth chamber are seeded murine cells from the blood brain barrier, to study the transport through the blood brain barrier.

Alternatively, Caco2 cells are seeded, as a model of the intestinal barrier.

By way of example, elastic membranes are selected in the group that comprises silicone rubber, e.g. polydimethylsiloxane (PDMS), fluorinated rubber, polyethylene terephthalate (PET) and polytetrafluoroethylene (PTFE) film; porous membranes are selected in the group that comprises silicone rubber, e.g. polydimethylsiloxane (PDMS), polycarbonate membrane, PTFE membrane.

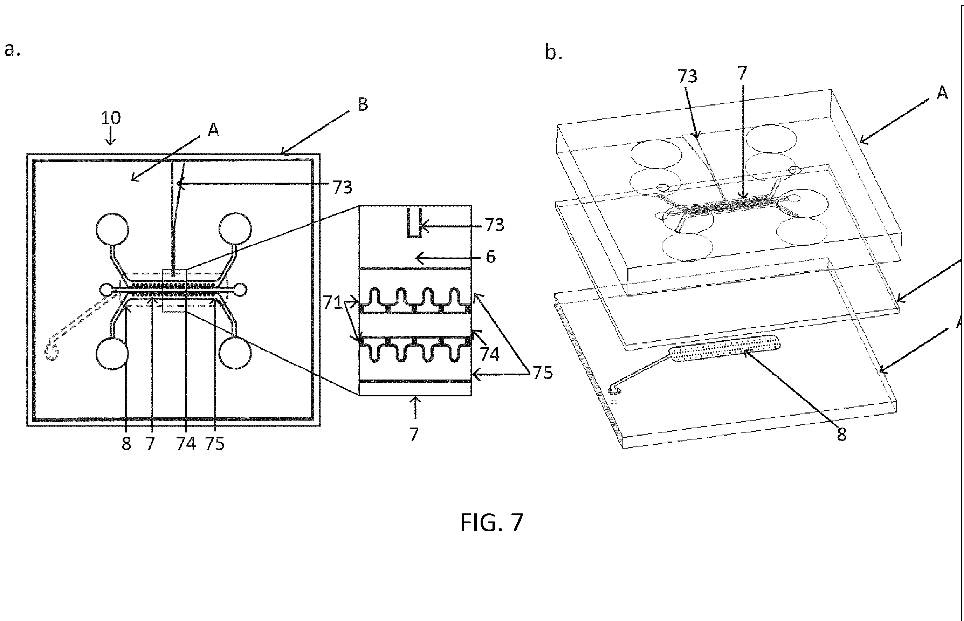

In a further embodiment, described in figure 7, said microfluidic device (10) comprises:
- two confinement means (A) that define at least one compartment that is a growth chamber (7) adapted to contain a cell matrix and at least one compartment that is an actuation chamber (8);
- a membrane (B) interposed between said two confinement means (A) that separates said growth chamber (7) from said collection chamber (8);
- guide (73) for housing and guiding the insertion of microelectrodes, where the volume of said guide is afforded inside said confinement means (A);
where said guide (73) is separated from said at least one growth chamber (7) by a wall (6), where said wall (6) is made of a material that is perforable and has a thickness in the range 100 µm - 5 mm, or 150 µm - 1.5 mm, or 200 µm - 1 mm.

Said guide (73) is positioned perpendicular to the central axis of the device. In this embodiment, the growth chamber (7) is comprised of a central microfluidic channel (74) delimited by two rows of T-shaped micropillars (71). The micropillars (71) are made attached to the upper wall of the upper compartment only, so as to leave them suspended and separated from the membrane. The central microfluidic channel (74) is surrounded by two side channels (75), intended to contain and transport the growth medium for feeding the cells.

Said embodiment is particularly suitable for studying the damages affecting the cartilage after a traumatic event.

Further subject matter of the present invention is a **kit for electrical measurements and/or stimulations in microfluidic conditions** that comprises:
a) the device (10) according to the present invention;
b) electrodes with a diameter comprised between about 50 µm and about 250µm, or between about 120 µm and about 230µm and a modulus of elasticity comprised between about 80 GPa and about 420 GPa, where said diameter and said modulus of elasticity preferably vary in an inverse way to one another, i.e. as the modulus of elasticity increases, the necessary diameter decreases.

By way of example, suitable electrodes are electrodes that have a diameter of 120 µm and a modulus of elasticity of about 200 GPa, or a diameter of 120 µm and modulus of elasticity of about 400 GPa, or a diameter of 250µm and a modulus of elasticity of about 80 GPa.

By way of example, said electrodes are selected in the group that comprises stainless steel pins, tungsten electrodes, coated in parylene C or not, silver wire.

Further subject matter of the present invention is a **method for electrical measurements and/or stimulations in microfluidic conditions** that comprises:
a) providing a kit according to the present invention;
b) embedding a cell matrix in said growth chamber (7) of said microfluidic device (10);
c) inserting through said at least one guide (1, 2, 3, 4) said at least one electrode (20) and exerting a pressure such as to perforate said wall (6) and reaching with said at least one electrode said growth chamber (7) and/or said collection chamber (9);
d) performing said electrical measurement and/or stimulation;
e) optionally, retracting said at least one electrode (20);
f) optionally, repeating said steps c) and/or d).

Advantageously, the present application offers a solution for precise direction and positioning of electrodes inside a growth chamber in a microfluidic device. This solution is made possible thanks to the thickness of the wall that separates the guide from the growth chamber and, optionally, of the support walls. Said thickness, perforable, guarantees stability, i.e. a precise and correct positioning of the electrode inserted in the growth chamber, and tightness, i.e. non leakage from/to the growth chamber. Solutions of the prior art, using walls of lower thickness, are suitable for chemical etching and do not offer as much stability and tightness. The fact that chemical etching is not applicable in the presence of walls with a thickness in the range here claimed should not be neglected. In fact, the etching known from the literature for this type of application is of isotropic type, therefore by chemical etching material in every direction is removed. Consequently, by way of example, a 150 µm high channel, housing the etching liquid, after removing a 30 µm thick wall, acquires a final height of 180 µm, with a consequent loss of resolution. To etch thicker walls, for example walls of at least 100 µm according to the present invention, this loss of resolution would become completely unacceptable. An additional problem to be faced applying chemical etching is due to the impossibility, after this kind of treatment, to impale directly a 3D cell matrix.

The present invention will now be further illustrated through the embodiments as provided below, in an illustrative and non-exhaustive way.

### Example 1: making of a microfluidic device according to one embodiment.

A multilayer microfluidic device (10) was made of silicone (polydimethylsiloxane, PDMS). Said microfluidic device comprises two compartments. Said compartments have a lower face, an upper face and four side faces. Said two compartments are placed on top of one another and are separated from one another by a membrane, where said membrane constitutes said lower face of said upper compartment and said upper face of said lower compartment. Said upper compartment is the growth chamber (7) and has the following dimensions: 1600 µm in width, 9 mm in length and 150 µm in height. Said lower compartment is the actuation chamber (8), used to provide cyclic mechanical stimulation, through pneumatic activation. The growth chamber (7) is comprised of a central microfluidic channel delimited by two rows of hexagonal micropillars (height 100 µm, side 28 µm, distance pillar-pillar 50 µm), equidistant from one another by 300 µm. The micropillars were made attached to the upper wall of the upper compartment only, so as to leave them suspended and separated from the membrane, at a distance of 50 µm. The central channel is surrounded by two side channels each of width 600 µm, intended to contain and transport the growth medium for feeding the cells. The device also comprises two guides (3, 4), the guide (4) positioned along the central axis of the device, the guide (3) at a distance of about 600 µm. Said guides have a length of 7.5 mm and are separated from said growth chamber (central channel and side channel, respectively) by a wall (6) with a thickness of about 200 µm.

The device is made through photo- and soft-lithography techniques. The layout of the device was performed through CAD (computer aided design) and comprises three layers (see Figure 3b): the first layer forms the growth chamber (7),150 µm in height (layer containing the suspended micropillars 100µm in height) and contains the aforesaid blind channels; the second layer comprises a flexible membrane; the third layer contains the mechanical actuation chamber (8), 50µm in height. The layout of the first and third layer was printed in high resolution (64.000 dpi) on a photomask and then transferred onto a silicon wafer through photolithography. For each of these layers, a photosensitive polymer film (negative photoresist, SU-8 2050), with thickness equal to the design height of such layer, was deposited on a silicon wafer having a diameter of 4" (about 10 cm). Such film was then exposed to collimated ultraviolet light (λ = 365 nm), filtered by the corresponding photomask, so as to induce the crosslinking of the polymer at the exposed zones only. After removing the excess non-polymerised photoresist, the three microstructured silicon wafers (i.e. having the layout of the bas-relief layers) were used as moulds for obtaining three blocks of PDMS. In particular, a solution of PDMS in the liquid phase was prepared by mixing its two components, pre-polymer and crosslinking, in a ratio of 10:1 and placed under vacuum for at least 15 minutes, so as to eliminate the presence of residual air. The solution was then poured into the moulds and left to crosslink at 65°C for 3 hours. The volume of PDMS to be poured into each mould was established so as to obtain the height of the desired block: in detail, 5 mm in the mould containing the micropillars, 500 µm in the non-microstructured mould to form the membrane and 500 µm in the mould of the actuation chamber.

The inlets to the various compartments were obtained by perforating the blocks of PDMS with punches of suitable dimensions at the designed access channels. In particular, two inlets of diameter 750 µm were made for the injection of the cell matrix, an inlet of diameter 500 µm for the pressurisation of the actuation, 4 inlets of diameter 5 mm for the injection of the growth medium. The assembly of the device was finally performed by gluing the three blocks of PDMS, subject to alignment, chemically and after plasma activation treatment.

### Example 2: generation and maturation of 3D cardiac constructs

The microfluidic device obtained as for example 1 was sterilised in an autoclave (20 min, 120°, 2 bar) and left in the oven at 80°C for 24 hours to restore the natural hydrophobicity of the PDMS. The microfluidic device was therefore used for the generation and maturation of 3D cardiac constructs, performed by loading a fibrin hydrogel solution with cardiac cells extracted from neonatal rat hearts or differentiated in cardiomyocytes starting from induced pluripotent cells. Before use, the actuation chamber (8) was completely filled with 1 µl of a buffer solution (phosphate buffered saline, or PBX 1X). In detail, the actuation chamber was connected with a flexible rubber hose (inner diameter 0.5 mm), half filled with PBS and half with air, to a compressed air line through a pressure regulator. For the filling of the actuation chamber with PBS, a constant pressure of 0.3 atm was applied for 45 minutes, so as to force the exit of air contained in the chamber through the PDMS walls themselves and their replacement with PBS.

In the case of neonatal rat cardiac cells, the cardiomyocytes were isolated through the following process. The cardiac tissue removed from the ventricles of neonatal rats was digested in trypsin (0.6 mg/ml, in Hank's Balanced Salt Solution, HBSS) for 18 hours. The digestion was then blocked by adding 10 ml of growth medium (Dulbecco Modified Eagle's Medium, DMEM) containing: 10% foetal bovine serum, 1% penicillin-streptomycin, 1% HEPES and 1% L-Glutamine. The isolation of the cells of the matrix was then obtained with two passages in collagenase solution (1 mg/ml in HBSS) at 37°C for 10 minutes. The cell suspension thus obtained was centrifuged at 700 rpm for 5 minutes, re-suspended in 25 ml of growth medium and seeded in a growth flask to isolate the cardiomyocyte fraction (non-adherent). After one hour, the fraction of non-adherent cells, comprising about 70% cardiomyocytes and 30% cardiac fibroblasts, was collected and centrifuged at 1,200 rpm for 5 minutes, in order to estimate the number of cells obtained.

In the event of using differentiated human cardiomyocytes from induced pluripotent stem cells, the cells are defrosted and prepared to be re-suspended in the fibrin gel. For the defrosting process the instructions provided by the manufacturer were followed.

For seeding inside the microfluidic device, the cardiac cells thus isolated were diluted in a fibrin gel solution at a cell concentration of 1 x 10⁵ cells/µl. In detail, for the seeding of 6 devices, 10 µl of fibrin gel were prepared by mixing 10 x 10⁵ cells (estimated volume of 4 µl), 2.5 µl of DMEM, 2 µl of fibrinogen (final concentration 20 mg/ml), 1 µl of aprotinin (stock concentration 16 TIU/ml in distilled water) and 0.5 µl of thrombin (final concentration 5 U/ml). The solution comprising fibrin gel and cells was aspirated through a micropipette and injected into each device (-1.2 µl per growth chamber). To obtain complete polymerisation of the fibrin gel, the devices were inserted in an incubator (T = 37°C, CO2 = 5%) for 5 minutes and then a growth medium containing a fraction of aminocaproic acid (2mg/ml) was injected in the side channels until completely covering the 4 compartments for the growth medium. Aminocaproic acid inhibits the digestion of fibrin gel by the cells. The growth medium was changed manually once a day throughout the whole incubation period (7 days).

Immediately after the polymerisation of the fibrin gel and the cell culture medium injection, the cell constructs were subjected to cyclic uniaxial mechanical stimulation (tensile deformation of about 10%, frequency of 1Hz), obtained by pressurising the salt solution contained in the actuation chamber, through an electronically driven solenoid valve (appointed to alternate the pressure in the chamber between environmental pressure and an overpressure of 0.5atm).

### Example 3: analysis of 3D cardiac constructs, recording of extracellular potential

To evaluate the spontaneous and synchronous beating of the cardiac construct left to mature following the application of mechanical stimulation, the electrical signal generated by the cells is measured. In detail, the two steel pins (120 µm diameter and 35 mm in length) which are the microelectrodes are inserted inside the two guides (3, 4) until reaching the wall (6) that separates said guides from the growth chamber (7). The microelectrodes perforate said wall (6) and are thus positioned in contact with the cardiac construct and with the growth medium. The final positioning can be performed by monitoring the insertion of the electrodes through a phase contrast microscope. A third electrode (reference electrode, 5) in silver/silver chloride is already housed in said microfluidic device, inside one of the wells of the growth medium. All the electrodes are connected to an amplification system (e.g. EXT-02 B extracellular amplifier by NPI ELECTRONIC) to amplify the signal 10,000 times. The output signal of the instrument is recorded through a signal acquisition board (e.g. NI USB-6211, National Instruments). Throughout the whole measurement process, the constructs are kept in the incubator or in an environmental chamber (T = 37°C, CO₂ = 5%).

### Example 4: use of the microfluidic device for screening pharmaceuticals

The mature cardiac construct generated inside the device, as described in Examples 2 and 3, can be used as an *in vitro* model for screening pharmaceuticals. The same experimental setup described in Example 3 was used to verify the effect of different pharmaceuticals on the spontaneous beating of the cardiac constructs. In detail, the effect of the following pharmaceuticals has been verified: 1) Sotalol, a non-selective beta-blocker that is used against cardiac arrhythmia and 2) Verapamil, a calcium antagonist compound that belongs to the class of type IV antiarrhythmic drugs. The pharmaceuticals were diluted in different concentrations in the growth medium and injected into the device from one of the openings intended for the inlet of the growth medium. Before performing the measurement, the pharmaceutical is incubated for 10 minutes. Specifically, for Verapamil the concentrations of 10 µM, 30 µM, 50 µM, 100 µM were used, and Sotalol at 3 µM, 7.5 µM, 15 µM, 30 µm and 60 µM was employed.

Before each test the control signal is recorded for each cardiac construct: the growth medium is substituted by a new medium and the new signal acquired after 10 minutes.

The curves obtained are analysed through software developed in MATLAB to evaluate the spontaneous contraction frequency of the construct.

Furthermore, characteristic parameters of the curves can be extrapolated: duration of the extracellular potential of the construct, depolarisation time of the construct, amplitude of the maximum peak of the construct and repolarisation time. With these measurements the chronotopical effects of the pharmaceuticals and/or their potential to cause arrhythmia (evaluation of the QT interval alteration) can be studied.

The suspension quantity of pharmaceutical used for each replicate is equal to 50 µl. The response of the constructs is evaluated 10 minutes after injection of the pharmaceutical.

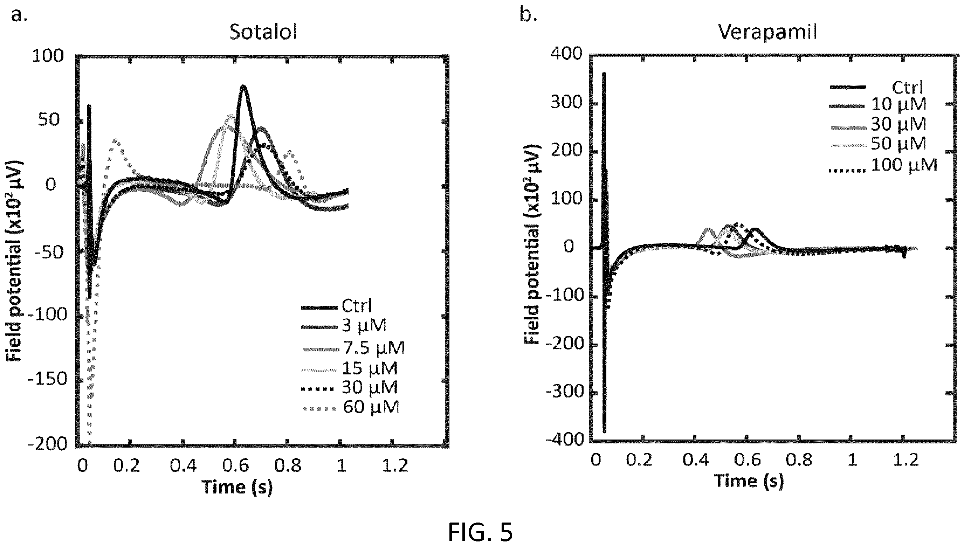

The results are displayed in figure 5 and show the variation of the extracellular potential of the construct treated with different pharmaceuticals using microtissues obtained from human cardiomyocytes derived from induced pluripotent stem cells. In particular, Sotalol induces longer repolarisation times of the construct (long QT), while Verapamil reduces the repolarisation time (short QT).

### Example 5: making of a microfluidic device according to a further embodiment.

A multilayer microfluidic device was made of silicone (polydimethylsiloxane, PDMS). The microfluidic device (10) is composed of three elements: an upper chamber, which is the growth chamber (7), size: 800 µm in width, 5 mm in length and 200 µm in height, a lower chamber that is the collection chamber (9), dimensions: 800 µm in width, 5 mm in length and 200 µm in height; a porous membrane (B) used for the cell culture. Said growth chamber (7) is positioned above said collection chamber (9). The layers are assembled so that the growth and collection chambers are orthogonal to each other and form a central exchange area (11) that is square shaped 800 µm x 800 µm. Guides (1, 2, 3, 4) 270 µm in width, 200 µm in height and 10mm in length are provided alongside the growth chamber, separated by a wall (6) of PDMS 1.5 mm thick. The four guides (1, 2, 3, 4) are oriented so that one of said guides is directed towards said central exchange area (11) and the other is about 3 mm from said central exchange area (11). The positioning of the guides is replicated with the same directions for the growth chamber (7) and for the collection chamber (9).

The device was produced through the use of photo- and soft-lithographic techniques like those described in example 1.

The assembly of the device was performed by gluing the growth chamber (7) and the collection chamber (9) to the membrane, interposed with the chambers themselves, using non-polymerised PDMS as glue ("stamp-and-stick bonding"). The microfluidic device was sterilised through the injection of ethanol (100%, for 10 minutes) and left in the oven at 80°C for 24 hours to restore the natural hydrophobicity of the PDMS. The device was then used for seeding murine cells from the blood-brain barrier. A solution of fibronectin in PBS (15 µg/ml) was injected into the growth and collection chambers. After 30 minutes of incubation, 100 µl of growth medium were injected into the growth and collection chambers and then 30 µl of a cell solution (5 million cells / ml) was injected into a single inlet of the growth chamber. Four electrodes were then inserted into the guides until perforating the first support wall of the electrode but not the wall (6) that separates from the growth chamber. Ideally, the electrodes directed towards the central exchange (11) are made of materials adapted for the measurement of voltage (silver/silver chloride) while the remaining electrodes are made of material adapted for the setting of current (platinum). The device was then kept in the incubator changing the medium every day.

### Example 6: transendothelial electrical resistance analysis

The microfluidic device loaded with the cell matrix as described in example 5 was used. To perform the transendothelial electrical resistance (TEER) measurements, the electrodes were inserted until perforating the wall of the growth chamber (7) and/or the collection chamber (9).

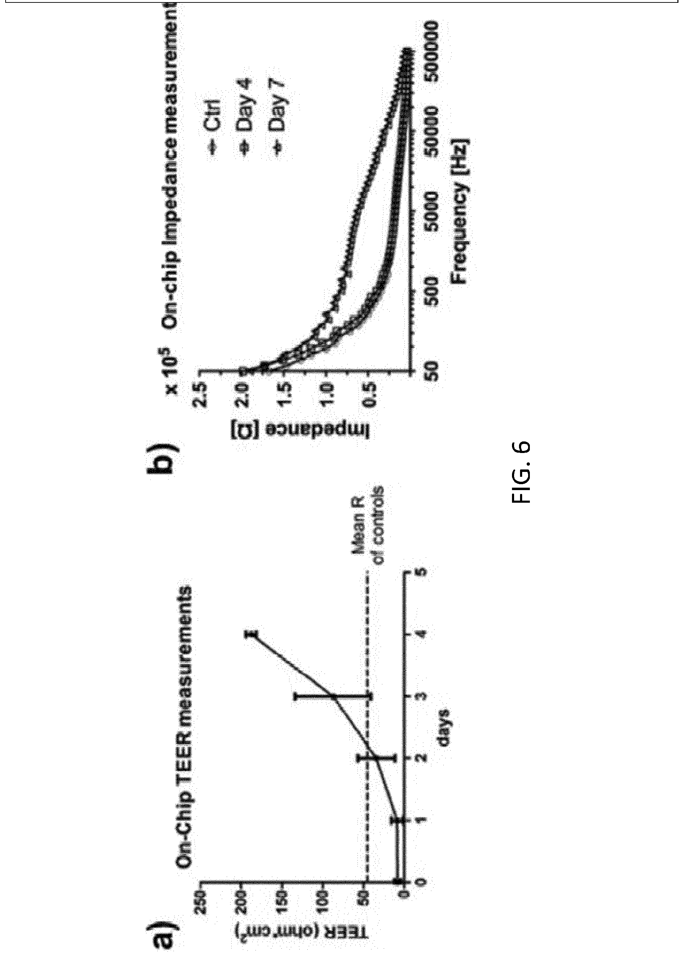

Subsequently, the electrodes were connected to a commercial measurement system (EVOM2) recording the TEER values on successive days of the cell culture. The results are provided in figure 6a and show an increasing trend of the TEER in the growth time. To obtain another type of reading, the electrodes were connected to an impedance meter, so as to measure impedance variations in the spectrum of the frequencies. These results, shown in figure 6b, show how impedance variations were detected up to 7 days of growing in the case in which the cells have not formed a compact cell monolayer (control), while an increase in impedance is highlighted after 7 days of culture when the cells create a compact cell monolayer typical of the blood-brain barrier (barrier).

### Example 7: making of a microfluidic device according to a further embodiment.

A multilayer microfluidic device (10) was made of silicone (polydimethylsiloxane, PDMS). Said microfluidic device comprises two compartments. Said compartments have a lower face, an upper face and four side faces. Said two compartments are placed on top of one another and are separated from one another by a membrane, where said membrane constitutes said lower face of said upper compartment and said upper face of said lower compartment. Said upper compartment is the growth chamber (7) and has the following dimensions: 1600 µm in width, 9 mm in length and 150 µm in height. Said lower compartment is the actuation chamber (8), used to provide cyclic mechanical stimulation, through pneumatic activation.

The growth chamber (7) is comprised of a central microfluidic channel (74) (300 µm wide) delimited by two rows of T-shaped micropillars (71) (height 105 µm, length 300 µm), with an interpillar gap of 30 µm. The micropillars (71) are made attached to the upper wall of the upper compartment only, so as to leave them suspended and separated from the membrane, at a distance of about 45 µm.

The central microfluidic channel (74) is surrounded by two side channels (75) each of width 600 µm, intended to contain and transport the growth medium for feeding the cells. The device also comprises one guide (73), positioned perpendicular to the central axis of the device. Said guide has a length of 7.5 mm and is separated from said growth chamber by a wall (6) with a thickness of about 100 µm.

The device is made through photo- and soft-lithography techniques. The layout of the device was performed through CAD (computer aided design) and comprises three layers (see Figure 7): the first layer comprises the growth chamber (7), 150 µm in height (layer containing the suspended micropillars 105µm in height) and contains the aforesaid guide (73); the second layer comprises a flexible membrane; the third layer comprises the mechanical actuation chamber (8), 50 µm in height. The device was printed and then assembled according to Example 1.

### Example 8: generation and maturation of 3D cartilage constructs

The microfluidic device in the embodiment described in example 7 was sterilised in an autoclave (20 min, 120°, 2 bar) and left in the oven at 80°C for 24 hours to restore the natural hydrophobicity of the PDMS. The microfluidic device was therefore used for the generation and maturation of 3D cartilage constructs. Before use, the actuation chamber (8) was filled with 1 µl of a buffer solution (phosphate buffered saline PBX 1X). In detail, the actuation chamber was connected with a flexible rubber hose (inner diameter 0.5 mm), half filled with PBS and half with air, to a compressed air line through a pressure regulator. For the filling of the actuation chamber with PBS, a constant pressure of 0.3 atm was applied for 45 minutes, so as to force the exit of air contained in the chamber through the PDMS walls themselves and their replacement with PBS.

Human primary articular chondrocytes are defrosted and expanded until the required number of cells is achieved. Cells are then harvested and re-suspended in the fibrin gel. For the defrosting and expansion processes the instructions provided by the manufacturer were followed.

For seeding inside the microfluidic device, the human primary articular chondrocytes were diluted in a fibrin gel solution at a cell concentration of 5 x 10⁴ cells/µl. In detail, for the seeding of 6 devices, 10 µl of fibrin gel were prepared by mixing 5 x 10⁵ cells (estimated volume of 4 µl), 2.5 µl of DMEM, 2 µl of fibrinogen (final concentration 20 mg/ml), 1 µl of aprotinin (stock concentration of 16 TIU/ml in distilled water) and 0.5 µl of thrombin (final concentration 5 U/ml). The solution comprising fibrin gel and cells was aspirated through a micropipette and injected into each device (-1.2 µl per growth chamber). To obtain complete polymerisation of the fibrin gel, the devices were inserted in an incubator (T = 37°C, CO₂ = 5%) for 5 minutes and then a chondrogenic differentiation medium containing a fraction of aminocaproic acid (2mg/ml) was injected in the side channels until completely covering the 4 compartments for the growth medium.

Composition of chondrogenic differentiation medium: DMEM containing 2% foetal bovine serum, 4.5 mg/ml d-glucose, 0.1 mM non-essential amino acids, 1 mM sodium pyruvate, 100 mM HEPES buffer, 100 U/ml penicillin, 100 µg/ml streptomycin and 0.29 mg/ml I-glutamine, and supplemented with 0.1 mM ascorbic acid 2-phosphate, 10 µg/ml insulin and 10 ng/ml TGF-β3.

Aminocaproic acid inhibits the digestion of fibrin gel by the cells. The chondrogenic differentiation medium was changed manually once every 3 days throughout the whole incubation period (14 days), until a stable human cartilage model is obtained.

### Example 9: induction of a localized tissue damage

The stable human cartilage model obtained as described in example 8 was used to generate a model of pathological cartilage state as post-traumatic event. In detail, with reference to figure 7, a steel pin (120 µm diameter and 35 mm in length) is inserted inside guide (73) until reaching the wall (6) that separates said guide from the growth chamber (7). The steel pin perforates said wall (6), enters the growth chamber until it reaches and breaks the cartilage microtissue. The resulting broken microtissue is exploited to generate pathological models, e.g. the traumatic cartilage damage.

## Claims

1. A **microfluidic device** (10) for the generation and/or cultivation and/or maturation and/or analysis of a cell matrix where said microfluidic device (10) comprises:
- confinement means (A) that defines at least one compartment that is a growth chamber (7) adapted to contain a cell matrix;
- at least one guide (1, 2, 3, 4, 73) for housing and guiding the insertion of microelectrodes, where the volume of said at least one guide is afforded inside said confinement means (A);
**characterised in that** said at least one guide (1, 2, 3, 4, 73) is separated from said at least one growth chamber (7) by a wall (6), where said wall (6) is made of a material that is perforable, with a thickness comprised in the range 100 µm - 5 mm, or 150 µm - 1.5 mm, or 200 µm - 1 mm.

2. The microfluidic device (10) according to claim 1 that further comprises at least one reference electrode (5) housed in at least one of said confinement means (A).

3. The microfluidic device (10) according to claim 1 or 2 that comprises:
- two confinement means (A) that define at least one compartment that is a growth chamber (7) adapted to contain a cell matrix and at least one compartment that is an actuation chamber (8) adapted to provide a mechanical stimulation through pneumatic activation;
- a membrane (B) interposed between said two confinement means (A), separating said growth chamber (7) from said actuation chamber (8).

4. The microfluidic device (10) according to claim 3, where said membrane is an elastic membrane.

5. The microfluidic device (10) according to claim 1 or 2 that comprises:
- two confinement means (A) that define at least one compartment that is a growth chamber (7) adapted to contain a cell matrix and at least one compartment that is a collection chamber (9);
- a membrane (B) interposed between said two confinement means (A) that separates said growth chamber (7) from said collection chamber (9).

6. The microfluidic device (10) according to claim 5, where said membrane is a porous membrane.

7. A **kit for electrical measurements and/or stimulations in microfluidic conditions** that comprises:
- the microfluidic device (10) according to one of claims from 1 to 6;
- electrodes with a diameter comprised between about 50 µm and about 250 µm, or between 120 and 230 µm, and a modulus of elasticity comprised between about 80 GPa and about 420 GPa.

8. A **method for electrical measurement and/or stimulation in microfluidic conditions** that comprises:
a) providing a kit according to claim 7;
b) embedding a cell matrix in said growth chamber (7) of said microfluidic device (10);
c) inserting through said at least one guide (1, 2, 3, 4) said at least one electrode (20) and exerting a pressure such as to surpass said wall (6) so as to reach with said at least one electrode (20) said growth chamber (7) and/or said collection chamber (9);
d) performing said electrical measurement and/or stimulation;
e) optionally, retracting said at least one electrode;
f) optionally, repeating said steps c) and/or d).

9. A **use** of a microfluidic device (10) according to one of claims from 1 to 6 for the generation and/or cultivation and/or maturation and/or analysis of a cell matrix.

10. The **use** of a microfluidic device (10) according to claim 5 for the measurement of the formation of endothelial/epithelial barriers *in vitro.*

11. The **use** of a microfluidic device (10) according to claim 3 for the measurement and/or induction of electrical activity of cell constructs *in vitro.*

12. The **use** of a microfluidic device (10) according to claim 3 for the evaluation of a traumatic cartilage damage.

## Patentansprüche

1. **Mikrofluidische Vorrichtung** (10) zur Erzeugung und/oder Kultivierung und/oder Reifung und/oder Analyse einer Zellmatrix, wobei die mikrofluidische Vorrichtung (10) umfasst:
- Begrenzungsmittel (A), die zumindest einen Bereich abgrenzen, welcher eine zur Aufnahme einer Zellmatrix geeignete Wachstumskammer (7) ist;
- zumindest eine Führung (1, 2, 3, 4, 73) zur Aufnahme und Führung des Einsetzens von Mikroelektroden, wobei das Volumen der zumindest einen Führung im Inneren der Begrenzungsmittel (A) vorgesehen ist;
**dadurch gekennzeichnet, dass** die zumindest eine Führung (1, 2, 3, 4, 73) von der zumindest einen Wachstumskammer (7) durch eine Wand (6) getrennt ist, wobei die Wand (6) aus einem perforierbaren Material mit einer Dicke im Bereich von 100 µm bis 5 mm, oder 150 µm bis 1,5 mm, oder 200 µm bis 1 mm besteht.

2. Mikrofluidische Vorrichtung (10) nach Anspruch 1, ferner umfassend zumindest eine Referenzelektrode (5), die in zumindest einem Begrenzungsmittel (A) untergebracht ist.

3. Mikrofluidische Vorrichtung (10) nach Anspruch 1 oder 2, umfassend:
- zwei Begrenzungsmittel (A), die zumindest einen Bereich, welcher eine zur Aufnahme einer Zellmatrix geeignete Wachstumskammer (7) ist, und zumindest einen Bereich, welcher eine zur mechanischen Stimulation durch pneumatische Ansteuerung geeignete Ansteuerungskammer (8) ist, abgrenzen;
- eine zwischen die zwei Begrenzungsmittel (A) eingebrachte Membran (B), die die Wachstumskammer (7) von der Ansteuerungskammer (8) trennt.

4. Mikrofluidische Vorrichtung (10) nach Anspruch 3, wobei die Membran eine elastische Membran ist.

5. Mikrofluidische Vorrichtung (10) nach Anspruch 1 oder 2, umfassend:
- zwei Begrenzungsmittel (A), die zumindest einen Bereich, welcher eine zur Aufnahme einer Zellmatrix geeignete Wachstumskammer (7) ist, und zumindest einen Bereich, welcher eine Sammelkammer (9) ist, abgrenzen;
- eine zwischen die zwei Begrenzungsmittel (A) eingebrachte Membran (B), die die Wachstumskammer (7) von der Sammelkammer (9) trennt.

6. Mikrofluidische Vorrichtung (10) nach Anspruch 5, wobei die Membran eine poröse Membran ist.

7. **Kit für elektrische Messungen und/oder Stimulationen unter mikrofluidischen Bedingungen** umfassend:
- die mikrofluidische Vorrichtung (10) nach einem der Ansprüche 1 bis 6;
- Elektroden mit einem Durchmesser von etwa 50 µm bis etwa 250 µm, oder von 120 bis 230 µm, und einem Elastizitätsmodul von etwa 80 GPa bis etwa 420 GPa.

8. **Verfahren zur elektrischen Messung und/oder Stimulation unter mikrofluidischen Bedingungen,** bei dem man
a) einen Kit nach Anspruch 7 bereitstellt;
b) eine Zellmatrix in die Wachstumskammer (7) der mikrofluidischen Vorrichtung (10) einbettet;
c) die zumindest eine Elektrode (20) durch die zumindest eine Führung (1, 2, 3, 4) einsetzt und Druck derart ausübt, dass die Wand (6) überwunden wird, um mit der zumindest einen Elektrode (20) die Wachstumskammer (7) und/oder die Sammelkammer (9) zu erreichen;
d) die elektrische Messung und/oder Stimulation durchführt;
e) optional, die zumindest eine Elektrode herauszieht;
f) optional, die Schritte c) und/oder d) wiederholt.

9. **Verwendung** einer mikrofluidischen Vorrichtung (10) nach einem der Ansprüche 1 bis 6 zur Erzeugung und/oder Kultivierung und/oder Reifung und/oder Analyse einer Zellmatrix.

10. **Verwendung** einer mikrofluidischen Vorrichtung (10) nach Anspruch 5 zur Messung der Bildung von endothelialen/epithelialen Barrieren *in vitro.*

11. **Verwendung** einer mikrofluidischen Vorrichtung (10) nach Anspruch 3 zur Messung und/oder Induktion von elektrischer Aktivität von Zellkonstrukten *in vitro.*

12. **Verwendung** einer mikrofluidischen Vorrichtung (10) nach Anspruch 3 zur Bewertung eines traumatischen Knorpelschadens.

## Revendications

1. Dispositif microfluidique (10) pour la génération et/ou la culture et/ou la maturation et/ou l'analyse d'une matrice de cellules où ledit dispositif microfluidique (10) comprend :
- un moyen de confinement (A) qui définit au moins un compartiment qui est une chambre de croissance (7) adaptée pour contenir une matrice de cellules ;
- au moins un guide (1, 2, 3, 4, 73) pour loger et guider l'insertion de microélectrodes, où le volume dudit au moins un guide est contenu à l'intérieur dudit moyen de confinement (A) ;
**caractérisé en ce que** ledit au moins un guide (1, 2, 3, 4, 73) est séparé de ladite au moins une chambre de croissance (7) par une paroi (6), où ladite paroi (6) est constituée d'un matériau qui est perforable, ayant une épaisseur comprise dans la plage de 100 µm à 5 mm, ou 150 µm à 1,5 mm, ou 200 µm à 1 mm.

2. Dispositif microfluidique (10) selon la revendication 1 qui comprend en outre au moins une électrode de référence (5) logée dans au moins l'un desdits moyens de confinement (A).

3. Dispositif microfluidique (10) selon la revendication 1 ou 2 qui comprend :
- deux moyens de confinement (A) qui définissent au moins un compartiment qui est une chambre de croissance (7) adaptée pour contenir une matrice de cellules et au moins un compartiment qui est une chambre d'actionnement (8) adaptée pour fournir une stimulation mécanique par activation pneumatique ;
- une membrane (B) intercalée entre lesdits deux moyens de confinement (A), séparant ladite chambre de croissance (7) de ladite chambre d'actionnement (8).

4. Dispositif microfluidique (10) selon la revendication 3, où ladite membrane est une membrane élastique.

5. Dispositif microfluidique (10) selon la revendication 1 ou 2 qui comprend :
- deux moyens de confinement (A) qui définissent au moins un compartiment qui est une chambre de croissance (7) adaptée pour contenir une matrice de cellules et au moins un compartiment qui est une chambre de collecte (9) ;
- une membrane (B) intercalée entre lesdits deux moyens de confinement (A) qui sépare ladite chambre de croissance (7) de ladite chambre de collecte (9).

6. Dispositif microfluidique (10) selon la revendication 5, où ladite membrane est une membrane poreuse.

7. Kit pour mesures et/ou stimulations électriques dans des conditions microfluidiques qui comprend :
- le dispositif microfluidique (10) selon l'une des revendications 1 à 6 ;
- des électrodes ayant un diamètre compris entre environ 50 µm et environ 250 µm, ou entre 120 et 230 µm, et un module d'élasticité compris entre environ 80 GPa et environ 420 GPa.

8. Procédé de mesure et/ou stimulation électrique dans des conditions microfluidiques qui comprend :
a) la fourniture d'un kit selon la revendication 7 ;
b) l'incorporation d'une matrice de cellules dans ladite chambre de croissance (7) dudit dispositif microfluidique (10) ;
c) l'insertion par l'intermédiaire dudit au moins un guide (1, 2, 3, 4) de ladite au moins une électrode (20) et l'application d'une pression de façon à dépasser ladite paroi (6) de façon à atteindre, avec ladite au moins une électrode (20), ladite chambre de croissance (7) et/ou ladite chambre de collecte (9) ;
d) la conduite de ladite mesure et/ou stimulation électrique ;
e) facultativement, le retrait de ladite au moins une électrode ;
f) facultativement, la répétition desdites étapes c) et/ou d).

9. Utilisation d'un dispositif microfluidique (10) selon l'une des revendications 1 à 6 pour la génération et/ou la culture et/ou la maturation et/ou l'analyse d'une matrice de cellules.

10. Utilisation d'un dispositif microfluidique (10) selon la revendication 5 pour la mesure de la formation de barrières endothéliales/épithéliales *in vitro.*

11. Utilisation d'un dispositif microfluidique (10) selon la revendication 3 pour la mesure et/ou l'induction de l'activité électrique de constructions de cellules *in vitro.*

12. Utilisation d'un dispositif microfluidique (10) selon la revendication 3 pour l'évaluation de dommages cartilagineux traumatiques.
